(19) Europäisches Patentamt
European Patent Office
Office européen des brevets

(11) **EP 3 236 874 B1**

(12) **FASCICULE DE BREVET EUROPEEN**

(45) Date de publication et mention de la délivrance du brevet:
**20.03.2019 Bulletin 2019/12**

(51) Int Cl.:
*A61B 90/10* (2016.01)     *A61B 34/20* (2016.01)
*A61B 90/00* (2016.01)

(21) Numéro de dépôt: **15823367.6**

(22) Date de dépôt: **22.12.2015**

(86) Numéro de dépôt international:
**PCT/FR2015/053735**

(87) Numéro de publication internationale:
**WO 2016/102898 (30.06.2016 Gazette 2016/26)**

(54) **SYSTEME D'ORIENTATION CHIRURGICAL**

**CHIRURGISCHES POSITIONIERUNGSSYSTEM**

**SURGICAL POSITIONING SYSTEM**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2014 FR 1463223**

(43) Date de publication de la demande:
**01.11.2017 Bulletin 2017/44**

(73) Titulaires:
• **Glard, Yann**
**13008 Marseille (FR)**
• **Pomero, Vincent**
**13120 Gardanne (FR)**

(72) Inventeurs:
• **Glard, Yann**
**13008 Marseille (FR)**
• **Pomero, Vincent**
**13120 Gardanne (FR)**

(74) Mandataire: **Santarelli**
**7, rue de la République**
**13001 Marseille (FR)**

(56) Documents cités:
EP-A1- 2 719 353      WO-A1-2006/109983
WO-A1-2014/176207     DE-T2- 69 534 862
KR-A- 20060 003 685

**Description**

La présente invention concerne un système d'orientation chirurgical et un ancillaire utilisable dans le cadre d'un tel système.

La structure squelettique interne d'un mammifère, humaine ou animale, est composée parfois d'une centaine d'os. La colonne vertébrale est une chaîne d'os ou de vertèbres permettant une certaine souplesse et de mouvement, tout en protégeant les structures nerveuses et vasculaires à l'intérieur et autour de la colonne vertébrale. La colonne vertébrale commence à la base du crâne, s'étend jusqu'au bassin et est composée de quatre régions - cervicale, thoracique, lombaire et pelvienne.

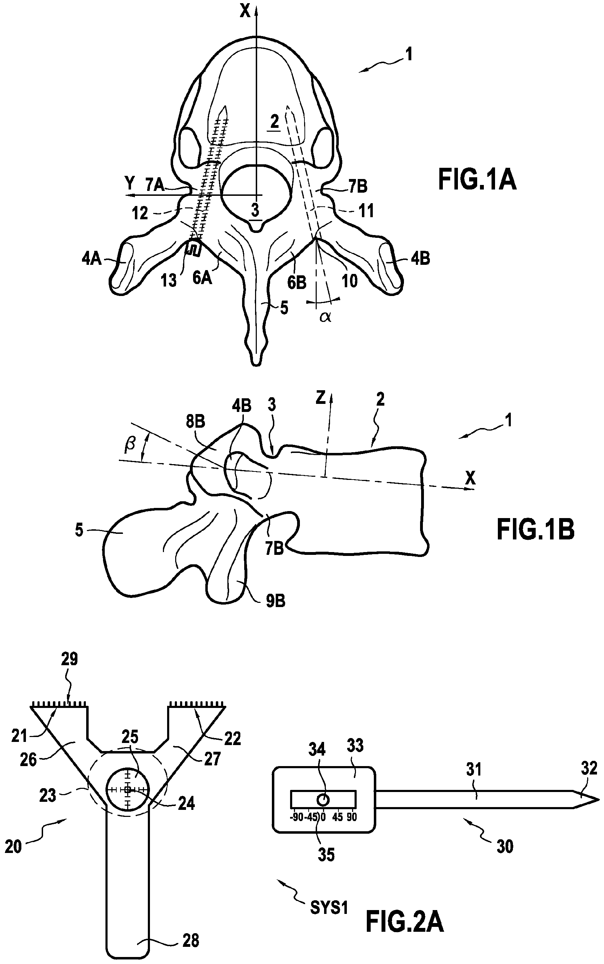

Les figures 1A, 1B représentent respectivement une vue de dessus et une vue de côté d'une vertèbre typique 1. La vertèbre 1 comprend : un corps vertébral 2 orienté vers l'avant ; un foramen vertébral 3 en forme de trou permettant à la moelle épinière de passer ; deux processus transversaux 4A, 4B, orientés vers l'arrière et vers l'extérieur ; un processus épineux 5 entre les processus transversaux 4A, 4B et orienté vers le bas ; deux lames 6A, 6B qui connectent les processus transversaux 4A, 4B au processus épineux 5 ; deux pédicules 7A, 7B qui connectent le corps vertébral 2 aux processus transversaux 4A, 4B ; deux facettes articulaires supérieures 8A (non représenté), 8B et deux facettes articulaires inférieures 9A (non représenté), 9B qui permettent l'articulation des vertèbres 1 entre-elles.

L'alignement vertébral normal ou idéal peut être perturbé suite à un traumatisme ou une maladie, par exemple la scoliose. Les vertèbres peuvent pivoter autour de trois axes (X, Y, Z), nécessitant parfois une intervention chirurgicale afin de corriger les anomalies et retrouver un alignement idéal ou, à tout le moins amélioré, de la colonne vertébrale.

Dans ce cas, au moins deux vertèbres 1 adjacentes sont généralement fusionnées l'une à l'autre par un procédé dans lequel un chirurgien ouvre le patient, généralement par le dos, détermine un point d'entrée 10 et perce des trous 11 dans les pédicules 7A, 7B des vertèbres 1. Les trous sont percés avec un angle axial alpha $\alpha$ (l'angle par rapport au plan XZ) et un angle sagittal beta $\beta$ (l'angle par rapport au plan XY), montré aux figures 1A, 1B respectivement.

Ensuite, des vis pédiculaires 12 comprenant des extrémités 13 en forme de U sont insérées dans les trous 11. (Pour des raisons de clarté de la figure 1A, un seul point d'entrée 10, trou 11, vis pédiculaire 12, et extrémité 13 sont montrés.) Les extrémités 13 reçoivent des éléments de liaison (non montrés), par exemple des barres, qui permettent de réduire la déformation et de fusionner les vertèbres 1 entre elles.

Ensuite, les éléments de liaison sont connectés entre les vis pédiculaires de deux vertèbres 1 adjacentes afin de corriger, au fur et à mesure, l'alignement de la colonne vertébrale, qui est basée sur des objectifs de correction approximatifs pour chaque niveau de la colonne vertébrale et sont dérivées à partir d'images médicales (rayons électromagnétiques 'X', tomodensitométrie, imagerie par résonnance magnétique, etc.) prises préopératoirement, c'est-à-dire avant une intervention chirurgicale. En conséquence, tous les trous 11 percés et les vis pédiculaires 12 placées dans les vertèbres 1 doivent être soigneusement positionnés et alignés afin de ne pas blesser les structures nerveuses et vasculaires adjacentes, voire provoquer la mort du patient. Tout au moins, il faudrait procéder à une seconde opération, entraînant des coûts et des risques supplémentaires.

Des systèmes de guidage ont été développés pour aider le chirurgien à percer les trous 11 dans la vertèbre 1 et placer précisément les vis pédiculaires 12.

L'article « Guided pedicle screw insertion and training » de Manbachi et al. décrit un système d'orientation chirurgical basé sur la navigation optoélectronique. La tomodensitométrie, ou TDM, est une technique d'imagerie médicale réalisée pendant une phase préopératoire afin de construire un modèle en trois dimensions de la zone à opérer, par exemple une ou plusieurs vertèbres. Pendant une phase peropératoire, c'est-à-dire l'opération proprement dit, des marqueurs optiques agencés sur les outils chirurgicaux sont captés par une pluralité de caméras qui filment la salle d'opération en temps réel, afin de repérer les positions des outils par rapport au modèle.

En outre, le patient est généralement allongé sur le dos pour la prise des images, tandis qu'il est allongé sur le ventre pour l'opération. En conséquence, les positions des vertèbres ne correspondent pas entre les images prises et la position du patient pendant l'opération.

Enfin, un tel système est couteux, encombrant dans une salle d'opération et peut manquer de précision si par exemple certains marqueurs sont obturés pendant l'opération. Dans le cas d'un système qui s'appuie sur de l'imagerie prise pendant la phase peropératoire, le personnel et le patient sont exposés aux rayons électromagnétiques (rayons X) et la durée de l'opération est plus longue.

Le brevet US 8,419,746 décrit un outil chirurgical comprenant une tige, au moins deux électrodes agencées sur la tige, et des moyens pour mesurer l'impédance entre les électrodes. Un changement d'impédance indique un vide, ce qui signifie que l'extrémité de la tige est sortie de l'os. Néanmoins, un tel outil ne permet pas au chirurgien de déterminer le trajet idéal, il ne fait que signaler un mauvais trajet a posteriori, signalement qui peut être trop tardif.

La demande de brevet WO 2006/109983 divulgue un premier dispositif posé sur des points de référence d'un pelvis afin d'indiquer un plan parallèle au pelvis pour l'insertion d'une cupule acétabulum.

Le brevet DE 695 34862 divulgue une pince fixée sur une projection osseuse et couplé à un cadre de référence comprenant des diodes électroluminescentes détectables par un système de caméra.

La demande de brevet WO 2014/176207 divulgue un dispositif d'enregistrement sur mesure qui est fabriqué à partir des images préopératoires et posé sur le patient lors de l'intervention chirurgicale.

La demande de brevet KR 2006 0003685 divulgue un premier dispositif posé sur trois points du pelvis et qui travaille en concertation avec un dispositif fixé sur le pelvis et un guide afin de déterminer les angles de l'un par rapport aux autres.

La demande de brevet EP 2 719 353 divulgue un procédé de fabrication d'un gabarit d'enregistrement ayant une surface qui se lie de manière précise à une surface d'un os sur un site cible chirurgical d'un patient pour une utilisation dans une chirurgie guidée par un système de navigation médicale.

En conséquence, il existe un besoin de systèmes et d'outils pour indiquer au préalable le bon trajet au chirurgien par rapport aux images prises préopératoirement et la position du patient pendant l'opération.

Des modes de réalisation de l'invention concernent un ancillaire chirurgical selon la revendication indépendante 1.

Selon un mode de réalisation, le premier point de contact, le deuxième point de contact, la zone de contact, le moyen de détermination et le moyen de communication sont intégrés dans une seule partie de l'ancillaire.

Selon un exemple, le premier point de contact, le deuxième point de contact et la zone de contact sont intégrés dans une première partie de l'ancillaire, et le moyen de détermination et le moyen de communication sont intégrés dans une deuxième partie qui puisse être encastrée sur la première partie.

Selon un mode de réalisation, la zone de contact est une zone tangentiel essentiellement plane.

Selon un mode de réalisation, le référentiel d'orientation de l'ancillaire permet un référentiel d'orientation de la zone opératoire d'être déterminé par rapport au référentiel d'orientation galiléen au moyen d'une matrice de rotation.

Selon un un exemple, l'ancillaire est en forme de compas et comprend :

- au moins deux branches aux extrémités inférieures desquelles le premier point et le deuxième point sont disposés ; et
- un troisième branche à l'extrémité inférieure de laquelle la zone de contact tangentiel en forme de plateau de palpation est disposée.

Selon l'invention, l'ancillaire est en forme de 'i-grec' et comprend :

- au moins deux branches aux extrémités desquelles le premier point et le deuxième point sont disposés en forme d'arrêtes rectilignes ;
- une troisième branche en forme de poignée ; et
- une zone centrale ayant une face inférieure qui forme la zone de contact tangentiel.

Selon un mode de réalisation, l'ancillaire comprend en outre des moyens de validation de la zone de contact tangentielle.

Selon un mode de réalisation, la zone de contact tangentielle est transparent et agencé d'une forme quadrillée et marquée afin de permettre un chirurgien de déterminer où exactement la zone tangentielle est en contact avec le troisième point de référence.

Selon un exemple, on peut ajuster la longueur, l'angle, et/ou l'inclinaison d'au moins une branche de l'ancillaire.

Selon un mode de réalisation, le premier point, le deuxième point, et la zone de contact tangentiel sont coplanaires.

Selon un exemple, le moyen de détermination du référentiel est un dispositif comprenant au moins l'un des composants suivants :

- un accéléromètre tri-axe ;
- un magnétomètre tri-axe ; et/ou
- un gyroscope tri-axe.

Selon un mode de réalisation, le moyen de détermination du référentiel d'orientation est un dispositif comprenant au moins trois marqueurs optiques non alignés, destinés à être visible par au moins une caméra filmant la zone opératoire.

Selon un mode de réalisation, le moyen de communication du référentiel d'orientation est un affichage visuel.

Selon un mode de réalisation, le moyen de communication du référentiel d'orientation est une liaison filaire ou non-filaire.

Des modes de réalisation de l'invention concernent en outre un ensemble comprenant au moins deux ancillaires selon l'invention, les ancillaires étant conçus pour des zones opératoires différentes l'une de l'autre.

Des modes de réalisation de l'invention concernent en outre un système d'orientation chirurgical comprenant au moins un ancillaire selon l'invention et un outil chirurgical comprenant :

- un moyen de détermination d'un référentiel d'orientation de l'outil ; et
- un moyen de communication du référentiel d'orientation de l'outil.

Selon un mode de réalisation, l'ancillaire et l'outil chirurgical sont capables d'être couplés à un même dispositif de détermination et de communication de référentiel d'orientation.

Des modes de réalisation de l'invention concernent en outre une salle opératoire équipée d'un ancillaire chirurgical selon l'invention et d'un dispositif d'affichage d'images et de traitement de données comprenant :

- un écran pour afficher des images prises de la zone opératoire ;
- un processeur ;
- des moyens d'entrée et de manipulation de données ; et
- des moyens de réception des données communiquées par l'ancillaire.

Des modes de réalisation de l'invention concernent en outre un procédé de préparation préopératoire d'une opération chirurgicale, comprenant les étapes de :

- prise d'au moins une image tridimensionnelle d'une zone opératoire ;
- déterminer au moins trois points de référence de la zone opératoire à partir de l'image tridimensionnelle ;
- calculer un référentiel opératoire au moyen des points de référence, le référentiel opératoire étant être identifié ultérieurement par un ancillaire selon l'invention ; et
- déterminer au moins un référentiel local pour le geste chirurgical à réaliser.

Des modes de réalisation de l'invention concernent en outre un support non transitoire lisible par un ordinateur et comprenant une programme d'instructions exécutable par ordinateur pour effectuer le procédé selon l'invention.

D'autres caractéristiques et avantages particuliers de la présente invention ressortiront de la description détaillée faite en référence aux figures dans lesquelles :

- les figures 1A, 1B, précédemment décrites, représentent respectivement une vue de dessus et une vue de côté d'une vertèbre typique,
- la figure 2A représente une vue de dessus d'un système d'orientation chirurgical comprenant un ancillaire chirurgical et un outil chirurgical selon un mode de réalisation,
- la figure 2B représente une vertèbre avec des points de référence pour l'opération,
- les figures 3A, 3B représentent respectivement une vue de dessus et une vue de côté de l'ancillaire chirurgical montré à la figure 2A en cours d'utilisation,
- la figure 4 représente un référentiel d'orientation d'une salle opératoire et un référentiel d'orientation d'une zone opératoire,
- les figures 5A, 5B représentent respectivement une vue de perspective et une vue de dessus d'un système d'orientation chirurgical selon un exemple,
- la figure 6 représente une vue de dessus d'un système d'orientation chirurgical selon un autre mode de réalisation,
- la figure 7 représente une vue de perspective d'un système d'orientation chirurgical selon un exemple,
- la figure 8 représente une vue de dessus d'un système d'orientation chirurgical selon un autre mode de réalisation,
- la figure 9 représente une salle opératoire équipée d'un système d'orientation chirurgical selon un mode de réalisation,
- la figure 10 représente un organigramme d'une phase préopératoire,
- la figure 11 représente un organigramme d'une phase peropératoire, et
- la figure 12 représente un support non transitoire lisible par un ordinateur et comprenant une programme d'instructions exécutable par ordinateur.

La figure 2A représente un système d'orientation chirurgical SYS1 selon un mode de réalisation. Le système SYS1 comprend un outil 20 (ci-après « ancillaire ») et un outil chirurgical 30, par exemple de forage.

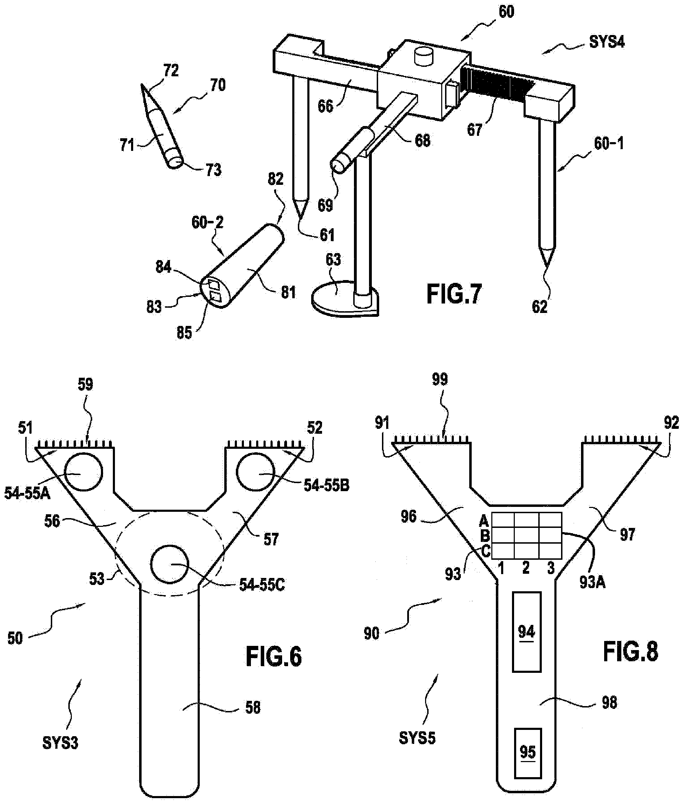

L'ancillaire 20 comprend : au moins deux pointes de contact 21, 22 ; une zone de contact tangentiel plane 23 ; un moyen de détermination 24 d'un référentiel d'orientation RA de l'ancillaire et un moyen de communication 25 du référentiel d'orientation RA de l'ancillaire. Le référentiel d'orientation RA de l'ancillaire 20 peut être assimilé à un référentiel d'orientation RO d'une zone opératoire ZO (par exemple la vertèbre 1) et permet en conséquence de connaître le référentiel d'orientation RO dans un référentiel d'orientation galiléen RS, par exemple d'une salle opératoire ZS (montrée à la figure 8). Dans ce qui suit, le terme référentiel d'orientation RO de la zone opératoire sera utilisé.

Dans ce mode de réalisation, l'ancillaire 20 est en forme de Y (« i-grec »), les points 21, 22 étant des arrêtes rectilignes d'une première branche 26 et d'une deuxième branche 27 respectivement, et une troisième branche 28 servant de poignée. Les branches 26, 27 sont les extrémités supérieures gauche et droite de l'Y respectivement, la branche 28 est l'extrémité inférieure centrale de l'Y, et la zone 23 est disposée au centre de l'Y et comprend une face inférieure plane (plus ou moins). L'ancillaire 20 comprend en outre des « picots » 29 sur les points 21, 22 qui empêchent l'ancillaire 20 de glisser une fois en contact avec la vertèbre. Les points 21, 22 et la zone de contact plane 23 sont coplanaires.

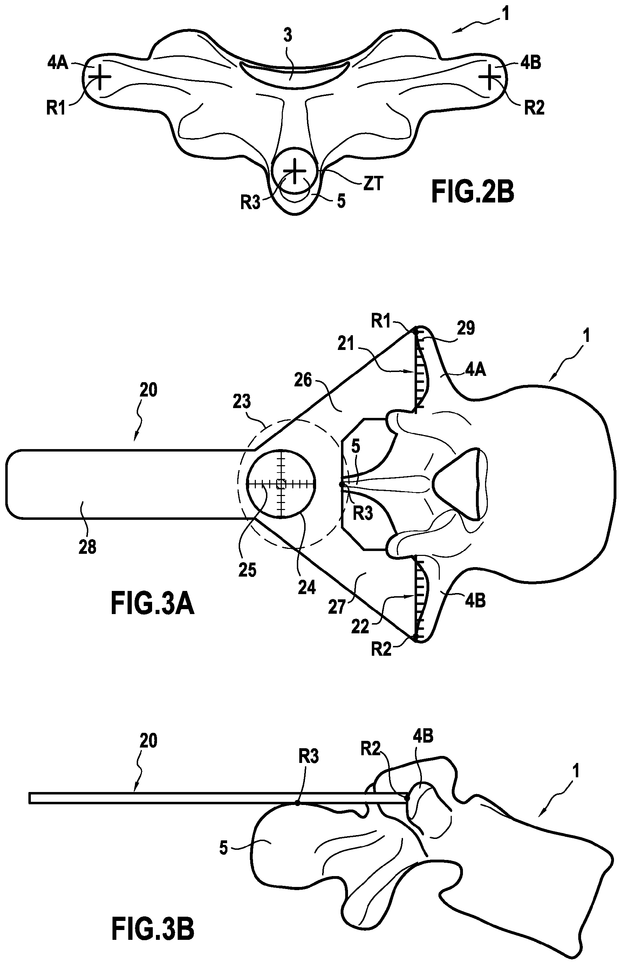

Comme montrée sur la figure 2B, la zone opératoire ZO (ici la vertèbre 1) comprend au moins trois points de référence R1, R2, R3. Dans le cas d'une vertèbre 1, les points R1, R2 sont disposés par exemple au sommet des processus transversaux 4A, 4B respectivement, et sont assez faciles à repérer à l'oeil nu, en particulier par un chirurgien expérimenté. Le point R3 est disposé sur le processus épineux 5 dans une zone tangentiel ZT, comme cela sera expliqué plus loin. En conséquence, les points de contact 21, 22 sont chacun destinés à venir en contact avec les points R1, R2 respectivement et la zone 23 est posée sur le point R3 afin de déterminer un système de coordonnées ou « référentiel d'orientation » RO de la zone opératoire ZO par rapport au référentiel d'orientation RS de la salle opératoire ZS.

L'outil chirurgical 30 est par exemple un outil pour percer la vertèbre 1, et comprend : une tige 31 ; un point 32 à l'extrémité devant de la tige ; une poignée 33 ; un moyen de détermination 34 d'un référentiel d'orientation RT de l'outil ; et un moyen de communication du référentiel d'orientation RT de l'outil.

Dans le cas le plus simple, les moyens de détermination 24, 34 sont des niveaux à bulle, aussi parfois appelés nivelle, et les moyens de communication 25, 36 sont des indicateurs visuels, par exemple des chiffres marqués autour du niveau à bulle ou même un simple cercle au centre du niveau.

Les figures 3A, 3B représentent respectivement une vue de dessus et une vue de côté de l'ancillaire 20 posé sur une vertèbre 1. Les dimensions de l'ancillaire 20 sont adaptées à l'application envisagée, par exemple environ 10 cm de largeur, 15 cm de longueur, et 0.50 cm d'épaisseur pour les opérations sur la colonne vertébrale humaine.

Dans une phase préopératoire, des images sont obtenues, par exemple de toute la colonne vertébrale, pour faire des reconstructions en trois-dimensions de la zone opératoire. Ensuite, pour chaque vertèbre, on détermine les points de référence R1, R2, R3, pour définir le référentiel d'orientation RO de la zone opératoire. Dans certains modes de réalisation, le point R3 est un point dans la zone tangentiel ZT, qui sera plus difficile à déterminer à l'oeil nu mais sera contacté par la zone de contact 23 par simple pose de l'ancillaire sur la zone ZT.

Ensuite, dans le cas de placement des vis pédiculaires, la saisie des directions de vissage optimal des vis pédiculaires 12 permet de définir un vecteur directeur pour chaque vis pédiculaire.

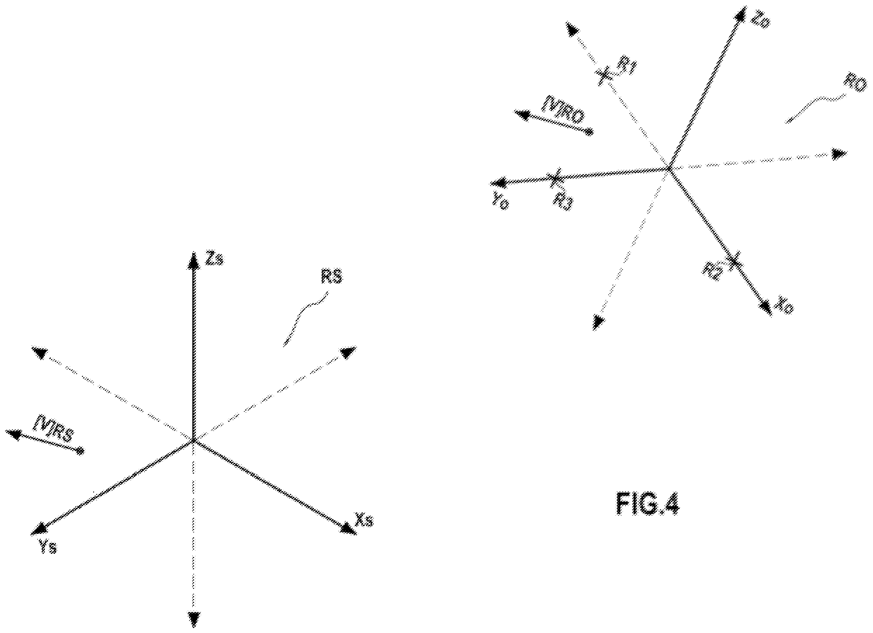

En référence à la figure 4, qui représente le référentiel d'orientation RO de la zone opératoire et le référentiel d'orientation galiléen RS de la salle opératoire, chaque référentiel d'orientation RO, RS comprend trois axes, Xo, Yo, Zo ; Xs, Ys, Zs respectivement. Une flèche [V]ro (ou « référentiel local ») représente un vecteur directeur V d'un geste chirurgical (par exemple la pose d'une vis pédiculaire) exprimée dans le référentiel d'orientation RO de la zone opératoire (actuellement dans la vertèbre). Le référentiel d'orientation RO de la zone opératoire n'est pas nécessairement aligné avec le référentiel RS de la salle d'opération, comme montré à la figure 4.

Ensuite, pendant la phase peropératoire, le chirurgien positionne l'ancillaire 20 sur la vertèbre 1, comme montré aux figures 3A, 3B, en mettant les points 21, 22 en contact aux points de référence R1, R2 respectivement, et ensuite posant la zone 23 sur le point de référence R3. Le référentiel d'orientation RO de la zone opératoire ZO est alors déterminé par rapport au référentiel RS de la salle opératoire, grâce aux moyens de détermination et de communication 24, 25 du référentiel d'orientation RO de la zone opératoire ZO.

Une matrice de rotation Mrors, qui exprime le référentiel d'orientation RO dans le référentiel d'orientation RS, est définie. Un vecteur directeur [V]rs dans le référentiel d'orientation RS de la salle opératoire ZS peut être établi par rapport au référentiel d'orientation RO de la zone opératoire ZO, précédemment établi, selon l'équation suivante :

$$[V]rs = Mrors \cdot [V]ro \qquad\qquad\qquad [\text{équation 1}]$$

Enfin, l'outil chirurgical 30 détermine et communique, grâce aux moyens de détermination et de communication 34, 35 du référentiel d'orientation RT de l'outil, en temps réel son orientation, notamment l'orientation de sa tige 31, dans le référentiel d'orientation RS de la salle opératoire. L'orientation dynamique de la tige 31 par rapport à l'orientation idéale de la vis pédiculaire à poser, permet au chirurgien d'adapter l'orientation de l'outil 30 pour la faire correspondre à l'orientation du vecteur directeur [V]ro exprimé dans le référentiel galiléen RS (soit [V]rs).

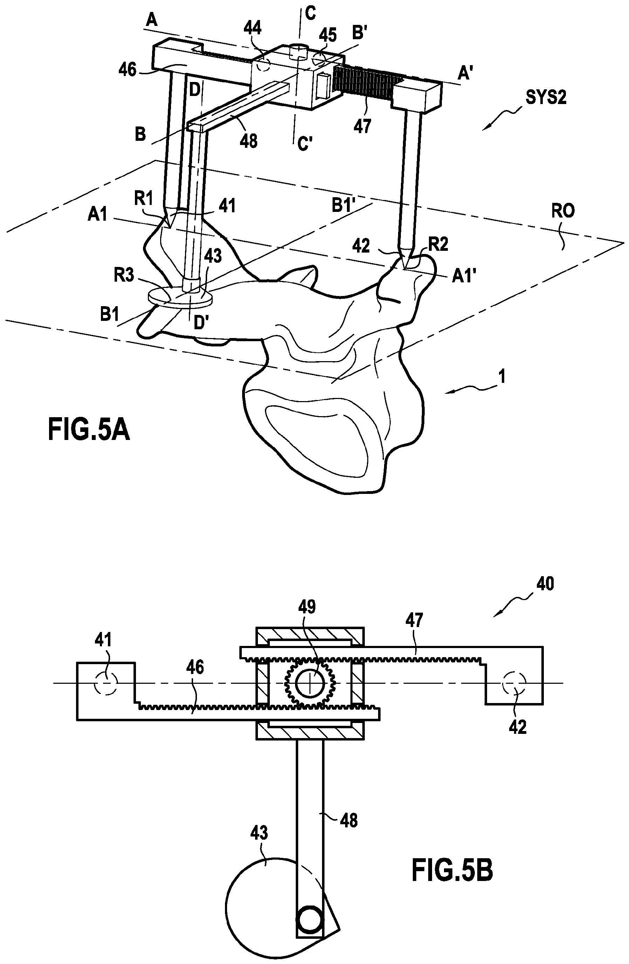

Les figures 5A, 5B représentent respectivement une vue en perspective et une vue de dessus d'un système d'orientation chirurgical SYS2 selon un exemple. Le système SYS2 comprend un ancillaire 40 et un outil chirurgical (non montré pour des raisons de simplicité). L'ancillaire 40 comprend : au moins deux points de contact 41, 42 ; une zone de contact tangentiel ou « plateau de palpation plane » 43 ; un moyen de détermination 44 du référentiel d'orientation RA (et en conséquence RO) et un moyen de communication 45 du référentiel d'orientation (non représentés en détail).

Dans ce mode de réalisation, l'ancillaire 40 est en forme d'un « compas », les points 41, 42 étant les extrémités inférieures des branches 46, 47 disposées selon un axe transversal A-A', et la zone de contact 43 étant disposée à l'extrémité inférieure d'une troisième branche 48 agencée le long d'un axe longitudinal B-B' qui bissecte l'axe A-A' à un axe vertical central C-C' et sert aussi de poignée.

Dans ce mode de réalisation, le moyen de détermination 44 est un système « MEMS » ou « microsystème électromécanique » qui détermine le plan de l'ancillaire. Ce moyen peut comprendre un accéléromètre tri-axe, un magnétomètre tri-axe, et/ou un gyroscope tri-axe, comme connu par l'homme du métier et ne sera pas expliqué plus en détail. Le moyen de communication 45 est une liaison filaire (câble) ou non-filaire (sans contact), par exemple par Wi-Fi ou Bluetooth.

Il sera noté que les vertèbres sur lesquelles on opère peuvent varier par rapport à la vertèbre « typique » montrée sur la figure 1, en fonction de la raison de l'opération, de l'âge du patient, de sa morphologie, etc. En conséquence, les points de référence R1, R2, R3 peuvent se trouver décalés par rapport aux normes. Dans ce cas, il est souhaitable de pouvoir ajuster l'ancillaire afin que les points de référence R1, R2, R3 puissent être contactés par l'ancillaire.

Dans ce mode de réalisation, les longueurs des branches 46, 47 peuvent être ajustées selon l'axe A'A' afin de tenir compte des variations possibles de la taille des vertèbres. A cette fin, l'ancillaire comprend un pignon 49 pour ajuster les longueurs des branches 46, 47, qui comportent des rails d'ajustement.

En outre, il sera noté que sur les figures 5A, 5B, la zone de contact 43 est articulée autour d'un axe vertical D-D' à l'extrémité distal de la branche 48.

Dans d'autres exemples, la longueur de la branche 48 peut être ajustée selon l'axe B-B' par exemple au moyen d'un système « télescopique », ainsi que les angles des branches 46, 47, 48 par rapport au centre, l'inclinaison des branches par rapport au plan formé par les axes A-A', B-B', etc.

A la figure 5A, l'ancillaire 40 est posée sur une vertèbre 1 en phase peropératoire. D'abord, les points 41, 42 des branches 47, 48 sont posés sur les points de référence R1, R2. Un axe A1-A1' est formé entre les points. Si nécessaire, la distance entre les points 41, 42 est modifiée par le pignon 49. Ensuite, l'ancillaire est pivoté autour de l'axe A1-A1' afin que la zone de contact 43 se pose sur le point R3. Les points 41, 42 et la zone de contact 43 sont alors coplanaires.

Un axe B1-B1' bissecte l'axe A1-A1' et la zone de contact 43. Le référentiel d'orientation RO de la zone opératoire ZO est déterminé par les moyens de détermination 44 et communiqué à l'extérieur de l'ancillaire 40 par les moyens de communication 45.

Une fois le référentiel d'orientation RO déterminé et communiqué, le chirurgien procède au perçage des trous à l'aide de l'outil chirurgical, qui peut être similaire à l'outil 30 décrit en relation avec la figure 2A, ou peut comporter des moyens de détermination MEMS et de communication filaire ou non-filaire (sans contact), comme décrit en relation avec l'ancillaire 40.

La figure 6 représente une vue de dessus d'un système d'orientation chirurgical SYS3 selon un autre mode de réalisation. Le système SYS3 comprend un ancillaire 50 et un outil chirurgical (non montré pour des raisons de simplicité).

L'ancillaire 50 comprend : au moins deux pointes de contact 51, 52 ; une zone de contact tangentiel 53 ; et des moyens de détermination et de communication 54-55A, 54-55B, 54-55C du référentiel d'orientation RA (RO). Les moyens 54-55A, 54-55B, 54-55C sont des marqueurs optiques non-alignés et destinés à être captés par une pluralité de caméras qui filment la salle d'opération ZS en temps réel, afin de repérer les positions des outils par rapport au modèle.

Similairement à l'ancillaire 20 décrit en relation avec la figure 2A, dans ce mode de réalisation, l'ancillaire 50 est en forme de Y (« i-grec »), les points 51, 52 étant des arrêtes rectilignes d'une première branche 56 et d'une deuxième branche 57 respectivement, et une troisième branche 58 servant de poignée. Les branches 56, 57 sont les extrémités supérieures gauche et droite de l'Y respectivement, la branche 58 est l'extrémité inférieure centrale de l'Y et la zone 53 est disposée au centre de l'Y. L'ancillaire 50 comprend en outre des « picots » 59 sur les points 51, 52 qui empêchent l'ancillaire 50 de glisser une fois en contact avec la vertèbre.

L'outil chirurgical peut être similaire à l'outil 30 décrit en relation avec la figure 2A (comprenant des nivelles), comporter des moyens de détermination MEMS et de communication filaire ou non-filaire (sans contact) ou même comporter des marqueurs optiques.

En outre, il n'est pas obligatoire que l'outil chirurgical comporte des tels moyens de détermination et de communication. Dans ce cas, il peut être un simple outil chirurgical classique.

La figure 7 représente une vue de perspective d'un système d'orientation chirurgical SYS4 selon un exemple. Le système SYS4 comprend un ancillaire 60 et un outil chirurgical 70. Dans cet exemple, l'ancillaire 60 est divisé en deux parties, une première partie 60-1 pour la prise de contact avec la zone opératoire, et une deuxième partie 60-2 de détermination et de communication du référentiel d'orientation de la salle opératoire.

La première partie 60-1 de l'ancillaire 60 est similaire à l'ancillaire 40 décrite en relation avec la figure 5A, et comprend : au moins deux points de contact 61, 62 ; une zone de contact tangentiel ou « plateau de palpation » 63 ; des branches 66, 67, 68 ; et une extrémité 69 pour recevoir la deuxième partie 60-2.

La deuxième partie 60-2 comprend : un corps 81 ; une extrémité devant 82 creuse pour recevoir l'extrémité derrière 69 de la première partie 60-1; un moyen de détermination 84 du référentiel d'orientation RA, RT (RO) de la partie 60-1 de l'ancillaire 60 ; et un moyen de communication 85 du référentiel d'orientation de l'ancillaire 60.

L'outil chirurgical 70 comprend : une tige 71 ; un point 72 à l'extrémité devant de la tige ; et une extrémité derrière 73.

De préférence, l'ancillaire 60 et l'outil chirurgical 70 coopèrent afin que la deuxième partie 60-2 de l'ancillaire 60 puisse être encastrée sur la première partie 60-1 et l'outil 70 d'une manière non définitive (elle peut être enlevée), précis (pas de jeu entre les éléments) et répétable. A cette fin, les extrémités derrières 69, 73 de la première partie 60-1 de l'ancillaire 60 et de l'outil chirurgical 70 respectivement peuvent comprendre des saillies reçus dans une encoche à l'intérieur de l'extrémité devant 82 creuse, obligeant la deuxième partie 60-2 d'être encastré d'une manière préalablement défini.

La deuxième partie 60-2 est d'abord encastrée sur l'extrémité 69 de la première partie 60-1 de l'ancillaire 60. Une fois le référentiel d'orientation RA déterminé et communiqué, la partie 60-1 est mise de côté et la partie 60-2 est enlevée et posée sur l'extrémité de l'outil chirurgical 70 pour déterminer et communiquer à nouveau le référentiel d'orientation RT de l'outil 70. Ce système permet une réduction de coût car un seul dispositif de détermination et de communication de référentiel est nécessaire, et peut être utilisé dans le cas où la zone opératoire n'est pas susceptible de changer de position en cours d'opération.

La figure 8 représente une vue de dessus d'un système d'orientation chirurgical SYS5 selon un autre mode de réalisation. Le système SYS5 comprend un ancillaire 90 et un outil chirurgical (non montré pour des raisons de simplicité).

L'ancillaire 90 comprend au moins deux pointes de contact 91, 92 ; une zone de contact tangentiel 93 ; des moyens de détermination 94 du référentiel d'orientation, par exemple un système « MEMS » comme décrit en relation avec la figure 5A, et des moyens de communication 95 du référentiel d'orientation par exemple une liaison non-filaire (sans contact).

Similairement à l'ancillaire 50 décrit en relation avec la figure 6, dans ce mode de réalisation, l'ancillaire 90 est en forme de Y (« i-grec »), les points 91, 92 étant des arrêtes rectilignes d'une première branche 96 et d'une deuxième branche 97 respectivement, et une troisième branche 98 servant de poignée. Les branches 96, 97 sont les extrémités supérieures gauche et droite de l'Y respectivement, la branche 98 est l'extrémité inférieure centrale de l'Y et la zone 93 est disposée au centre de l'Y. L'ancillaire 90 comprend en outre des « picots » 99 sur les points 91, 92 qui empêchent l'ancillaire 90 de glisser une fois en contact avec la vertèbre.

L'ancillaire 90 comprend en outre des moyens de validation 93A de la zone de contact tangentielle 93. A cette fin, dans ce mode de réalisation, la zone 93 est transparent et agencé d'une forme quadrillée et de préférence marquée par exemple par des chiffres (1 à 3) et des lettres (A à C), afin de permettre un chirurgien de déterminer où exactement la zone tangentielle est en contact avec le troisième point de référence. Par exemple, pendant la phase préopératoire, il peut être déterminé que le troisième point de référence doit être en contact avec la zone A3 de la zone tangentielle. Les picots 99 peuvent être aussi équipés de marqueurs (non-montrés) afin de faciliter le repérage des points de contact.

Dans une autre mode de réalisation, les moyens de validation sont une zone de contact sensible qui détecte le contact avec le troisième point de référence, et le communique par exemple par les moyens de communication.

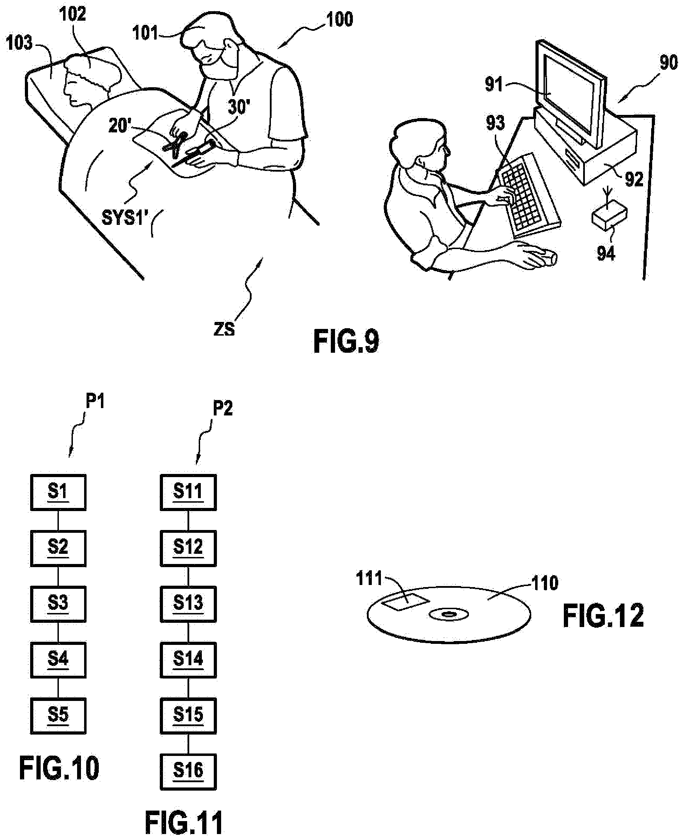

La figure 9 représente une salle opératoire ZS équipée d'un système d'orientation chirurgical. A titre d'exemple, un système SYS1' est montré ici, comprenant un ancillaire 20' et un outil 30' équipés par des moyens de détermination « MEMS » et de moyens de communication non-filaire.

La salle opératoire ZS est équipée d'un dispositif 90 d'affichage d'images et de traitement de données, tel un ordinateur. Le dispositif 90 comprend un écran 91, un processeur 92, des moyens d'entrée et de manipulation de données 93 (un clavier, un souris, un capteur de voix, etc.), et des moyens de réception 94 des données communiquées par l'ancillaire 20' et/ou l'outil 30'.

La salle opératoire comprend en outre un « entité opératoire » 100 comprenant un chirurgien 101 qui opère sur un patient 102 allongé sur une table d'opération 103.

L'écran 91 permet d'afficher des images I obtenues de la zone opératoire ZO pendant la phase préopératoire. Le personnel du bloc opératoire, et particulièrement le chirurgien, peut consulter les images pendant l'opération. Ces images peuvent être « statiques » ou avantageusement « dynamiques ». Par dynamique, il est entendu que les référentiels de l'ancillaire 20' et/ou de l'outil chirurgical 30' sont déterminés, communiqués à l'ordinateur 90, et affichés sur l'écran 91 en temps réel. Le chirurgien 101 peut alors avoir une idée précise de l'orientation de ses outils par rapport à la vertèbre.

Dans un mode de réalisation, le système est interactif et permet au chirurgien 101 de donner des instructions orales, par exemple « Affichez la vertèbre L5. » afin que l'ordinateur affiche l'image correspondant à la vertèbre L5.

La figure 10 représente un organigramme d'une phase préopératoire P1, et la figure 11 représente un organigramme d'une phase peropératoire P2.

La phase P1 comprend les étapes S1 à S5. A l'étape S1, une image I d'au moins une zone opératoire ZO est prise, par exemple par un moyen de tomodensitométrie. A l'étape S2, au moins trois points de référence R1, R2, R3 sont déterminés et enregistrés dans le cas d'un système dynamique, ou simplement notés dans le cas d'un système statique. A l'étape S3, un référentiel opératoire RO, comme décrit plus haut en relation avec la figure 4, est calculé au moyen des points de référence et ensuite enregistré ou noté. A l'étape S4, au moins un vecteur directeur [V]ro est déterminé pour le geste chirurgical à réaliser et ensuite enregistré ou noté. A l'étape S5, le procédé est répété si nécessaire pour d'autres zones opératoires.

La phase P2 comprend les étapes S11 à S16. A l'étape S11, la zone opératoire ZO est exposée. A l'étape S12, un point de l'ancillaire est posé sur le premier point de référence R1. A l'étape S13, un autre point de l'ancillaire est posée sur le deuxième point de référence R2. A l'étape S14, la zone de contact de l'ancillaire est posé sur le troisième point de référence R3. A l'étape S15, le référentiel d'orientation RO de la zone opératoire ZO est déterminé et communiqué, permettant le calcul de la matrice de rotation Mrors et du vecteur directeur [V]rs. A l'étape S16, l'outil chirurgical est utilisé pour réaliser un geste chirurgical selon le vecteur directeur calculé.

La figure 12 représente un support non transitoire 110 lisible par un ordinateur et comprenant une programme d'instructions 11 exécutable par ordinateur. Le programme d'instructions peut comprendre l'algorithme de calcul décrit en relation avec la figure 4.

Des modes de réalisation concernent en outre un ensemble ou « kit » d'au moins deux ancillaires 20, 20', 50, chaque ancillaire étant conçue pour des zones opératoires ZO différentes l'une l'autre, par exemple ayant des dimensions différentes, des angles différentes entre les branches, etc. Ceci permet de couvrir une gamme de variations anatomiques. Dans un mode de réalisation, les ancillaires 20, 20', 50 ont différent tailles, par exemple petit, moyen, et large.

Il sera compris par l'homme du métier que les modes de réalisation décrits en ce qui précède peuvent être modifiés.

Par exemple, les moyens de communication 25, 35, 45, 55, 65 peuvent être un écran numérique, des diodes électroluminescentes DEL (« LED » ou « Light-Emitting Diode » en anglais) par exemple vertes, oranges et rouges qui s'allument, des liaisons filaires (un câble connecté à l'outil chirurgical ou au dispositif de traitement de données), non-filaires (Wi-Fi, NFC, Bluetooth, etc), un signal auditif et, plus généralement, tout moyen de communication d'information.

En ce qui précède, les zones de contact 23, 43, 53, 63 ont été décrites comme des zones essentiellement planes qui se posent sur une zone de contact tangentiel ZT. (Par « essentiellement planes » il est compris que la zone est plus ou moins planaire dans les limites de fabrication). Néanmoins, il sera compris par l'homme du métier que ces zones de contact peuvent avoir toute d'autre forme conçue pour venir en contact avec une zone déterminée. Par exemple, ils peuvent être concaves pour se poser sur une forme arrondie, telle que les processus épineux 5, convexes pour se poser dans une dépression, etc.

Dans un mode de réalisation, non montré, un outil de détermination et de communication d'un référentiel d'orientation est fixé sur la zone opératoire ZO elle-même afin de vérifier en permanence sa position, par exemple pour s'assurer que le patient n'a pas bougé pendant l'opération, pour les opérations très délicates.

La position du patient, et plus particulièrement de la zone opératoire, peut être ajustée jusqu'à ce que la bonne orientation soit trouvée. Des moyens (des sangles, des pinces, etc.) pour tenir la zone opératoire (le patient) dans une position donnée peuvent être mis en oeuvre, soit avant l'opération, soit pendant l'opération.

Il sera compris par l'homme du métier que certains éléments décrits en relation avec un mode de réalisation (par exemple les moyens de détermination et de communication, les « MEMS », les picots, etc.) peuvent être appliqués aux autres modes de réalisation.

L'outil chirurgical est par exemple un perforateur, un tournevis et, en général, tout outil qui permet de réaliser un acte chirurgical.

Les matériaux utilisés pour l'ancillaire et l'outil chirurgical peuvent, de préférence, être stérilisés et ne posent pas de problème de biocompatibilité.

Comme évoqué plus haut, dans certains modes de réalisation, il n'est pas obligatoire que l'outil chirurgical soit équipé d'un moyen de détermination et de communication de position. Dans certains cas, une fois que le référentiel d'orientation RO a été obtenu, le chirurgien peut facilement déterminer lui-même l'angle correct, par exemple un angle de 90° par rapport au référentiel d'orientation de l'ancillaire.

Dans certains modes de réalisation, les branches de l'ancillaire 20, 20', 50 peuvent être articulées autour de la zone centrale, par exemple aux moyens des charnières disposés entre la zone centrale et chaque branche.

Enfin, d'autres modes de calcul des vecteurs directeurs peuvent être mises en oeuvre.


## Revendications

**1.** Ancillaire (20, 20' ; 50 ; 90) chirurgical comprenant au moins :

- un premier point de contact (21 ; 51 ; 91) destinée à venir en contact avec un premier point de référence (R1) d'une zone opératoire (ZO) ;

- un deuxième point de contact (22 ; 52 ; 92) destinée à venir en contact avec un deuxième point de référence (R2) de la zone opératoire ;
- une zone de contact (23 ; 53 ; 93) tangentielle destinée à venir en contact avec un troisième point de référence (R3) de la zone opératoire ;
- un moyen de détermination (24 ; 54A, 54B, 54C ; 84 ; 94) d'un référentiel d'orientation (RA) de l'ancillaire dans un référentiel d'orientation galiléen (RS) ; et
- un moyen de communication (25 ; 55A, 55B, 55C ; 85 ; 95) du référentiel d'orientation déterminé

**caractérisé en ce que** l'ancillaire est en forme de i-grec (Y) et comprend :

- au moins deux branches (26, 27 ; 56, 57 ; 96, 97) aux extrémités desquelles le premier point et le deuxième point (21, 22 ; 51, 52 ; 91, 92) sont disposés en forme d'arêtes rectilignes ;
- une troisième branche (28 ; 58 ; 98) en forme de poignée ; et
- une zone centrale ayant une face inférieure qui forme la zone de contact tangentielle (23 ; 53 ; 93).

2. Ancillaire (20, 20' ; 50 ; 90) selon la revendication 1, dans lequel le premier point de contact (21 ; 51 ; 91), le deuxième point de contact (22 ; 52 ; 92), la zone de contact (23 ; 53 ; 93), le moyen de détermination (24 ; 54A, 54B, 54C ; 94) et le moyen de communication (25 ; 55A, 55B, 55C ; 95) sont intégrés dans une seule partie de l'ancillaire.

3. Ancillaire (20, 20' ; 50 ; 90) selon l'une des revendications 1 ou 2, dans lequel la zone de contact (23 ; 53 ; 93) est une zone tangentiel plane.

4. Ancillaire (20, 20' ; 50 ; 90) selon l'une des revendications 1 à 3, dans lequel le référentiel d'orientation (RA) de l'ancillaire permet un référentiel d'orientation (RO) de la zone opératoire (Z0) d'être déterminé par rapport au référentiel d'orientation galiléen (RS) au moyen d'une matrice de rotation (Mrors).

5. Ancillaire (90) selon la revendication 1, comprenant en outre des moyens de validation (93A) de la zone de contact tangentielle (93).

6. Ancillaire (90) selon la revendication 5, dans lequel la zone de contact tangentielle (93) est transparent et agencé d'une forme quadrillée et marquée afin de permettre un chirurgien de déterminer où exactement la zone tangentielle est en contact avec le troisième point de référence.

7. Ancillaire (20, 20' ; 50 ; 90) selon l'une des revendications 1 à 6, dans lequel le premier point (21 ; 51 ; 91), le deuxième point (22 ; 52 ; 92), et la zone de contact tangentiel (23 ; 53 ; 93) sont coplanaires.

8. Ancillaire (20' ; 50 ; 90) selon l'une des revendications 1 à 7, dans lequel le moyen de détermination (24 ; 84 ; 94) du référentiel est un dispositif comprenant au moins l'un des composants suivants :

- un accéléromètre tri-axe ;
- un magnétomètre tri-axe ; et/ou
- un gyroscope tri-axe.

9. Ancillaire (50) selon l'une des revendications 1 à 8, dans lequel le moyen de détermination (54A, 54B, 54C) du référentiel d'orientation est un dispositif comprenant au moins trois marqueurs optiques non alignés, destinés à être visible par au moins une caméra filmant la zone opératoire.

10. Ancillaire (20) selon l'une des revendications 1 à 9, dans lequel le moyen de communication (25) du référentiel d'orientation est un affichage visuel.

11. Ancillaire (50 ; 90) selon l'une des revendications 1 à 10, dans lequel le moyen de communication (55A, 55B, 55C ; 85 ; 95) du référentiel d'orientation est une liaison filaire ou non-filaire.

12. Un ensemble comprenant au moins deux ancillaires (20, 20' ; 50) selon la revendication 1, les ancillaires étant conçus pour des zones opératoires (ZO) différentes l'une de l'autre.

13. Système d'orientation chirurgical (SYS1, SYS1' ; SYS3) comprenant au moins un ancillaire (20, 20'; 50) selon l'une des revendications 1 à 11 et un outil chirurgical (30 ; 70) comprenant :

- un moyen de détermination (34 ; 84) d'un référentiel d'orientation (RT) de l'outil ; et
- un moyen de communication (35 ; 85) du référentiel d'orientation (RT) de l'outil.

**14.** Salle opératoire (ZS) équipée d'un ancillaire (20, 20' ; 40 ; 50 ; 60 ; 90) chirurgical selon l'une des revendications 1 à 11 et d'un dispositif d'affichage d'images et de traitement de données (90) comprenant :

- un écran (91) pour afficher des images (I) prises de la zone opératoire (ZO) ;
- un processeur (92) ;
- des moyens d'entrée et de manipulation de données (93) ; et
- des moyens de réception (94) des données communiquées par l'ancillaire.

**15.** Procédé de préparation préopératoire (P1) d'une opération chirurgicale, comprenant les étapes de :

- prise (S1) d'au moins une image (I) tridimensionnelle d'une zone opératoire (ZO) ;
- déterminer (S2) au moins trois points de référence (R1, R2, R3) de la zone opératoire à partir de l'image tridimensionnelle ;
- calculer (S3) un référentiel opératoire (RO) au moyen des points de référence, le référentiel opératoire étant être identifié ultérieurement par un ancillaire selon l'une des revendications 1 à 11 ; et
- déterminer (S4) au moins un référentiel local ([V]ro) pour le geste chirurgical à réaliser.

**16.** Support non transitoire (110) lisible par un ordinateur et comprenant une programme d'instructions (11) exécutable par ordinateur pour effecteur le procédé selon la revendication 15.

**Patentansprüche**

**1.** Chirurgische Hilfsvorrichtung (20, 20'; 50; 90), zumindest enthaltend:

- einen ersten Kontaktpunkt (21; 51; 91), der dazu bestimmt ist, mit einem ersten Bezugspunkt (R1) eines Operationsbereichs (ZO) in Kontakt zu gelangen;
- einen zweiten Kontaktpunkt (22; 52; 92), der dazu bestimmt ist, mit einem zweiten Bezugspunkt (R2) des Operationsbereichs in Kontakt zu gelangen;
- einen tangentialen Kontaktbereich (23; 53; 93), der dazu bestimmt ist, mit einem dritten Bezugspunkt (R3) des Operationsbereichs in Kontakt zu gelangen;
- eine Ermittlungseinrichtung (24; 54A, 54B, 54C; 84; 94) zum Ermitteln eines Ausrichtungsbezugssystems (RA) der Hilfsvorrichtung in einem Ausrichtungsinwertialsystem (RS); und
- eine Kommunikationseinrichtung (25; 55A, 55B, 55C; 85; 95) für das ermittelte Ausrichtungsbezugssystem,

**dadurch gekennzeichnet, dass**
die Hilfsvorrichtung Ypsilon-förmig (Y) ist und enthält:

- zumindest zwei Schenkel (26, 27; 56, 57; 96, 97), an deren Enden der erste Punkt und der zweite Punkt (21, 22; 51, 52; 91, 92) in Form von geradlinigen Kanten angeordnet sind;
- einen dritten Schenkel (28; 58; 98) in Form eines Griffs; und
- einen Mittelbereich mit einer Unterseite, der den tangentialen Kontaktbereich (23; 53; 93) bildet.

**2.** Hilfsvorrichtung (20, 20'; 50; 90) nach Anspruch 1, wobei der erste Kontaktpunkt (21; 51; 91), der zweite Kontaktpunkt (22; 52; 92), der Kontaktbereich (23; 53; 93), die Ermittlungseinrichtung (24; 54A, 54B, 54C; 94) und die Kommunikationseinrichtung (25; 55A, 55B, 55C; 95) in einem einzelnen Teil der Hilfsvorrichtung integriert sind.

**3.** Hilfsvorrichtung (20, 20'; 50; 90) nach einem der Ansprüche 1 oder 2, wobei der Kontaktbereich (23; 53; 93) ein flacher, tangentialer Bereich ist.

**4.** Hilfsvorrichtung (20, 20'; 50; 90) nach einem der Ansprüche 1 bis 3, wobei mit dem Ausrichtungsbezugssystem (RA) der Hilfsvorrichtung ein Ausrichtungsbezugssystem (RO) des Operationsbereichs (ZO) bezüglich des Ausrichtungsinertialsystems (RS) mittels einer Rotationsmatrix (Mrors) ermittelt werden kann.

**5.** Hilfsvorrichtung (90) nach Anspruch 1, ferner enthaltend eine Validierungseinrichtung (93A) für den tangentialen Kontaktbereich (93).

6. Hilfsvorrichtung (90) nach Anspruch 5, wobei der tangentiale Kontaktbereich (93) transparent ausgeführt und in einem Gittermuster angeordnet und markiert ist, damit ein Chirurg ermitteln kann, wo genau der tangentiale Bereich mit dem dritten Bezugspunkt in Kontakt ist.

7. Hilfsvorrichtung (20, 20'; 50; 90) nach einem der Ansprüche 1 bis 6, wobei der erste Punkt (21; 51; 91), der zweite Punkt (22; 52; 92) und der tangentiale Kontaktbereich (23; 53; 93) koplanar verlaufen.

8. Hilfsvorrichtung (20'; 50; 90) nach einem der Ansprüche 1 bis 7, wobei die Ermittlungseinrichtung (24; 84; 94) zum Ermitteln des Bezugssystems eine Vorrichtung ist, die zumindest eine der nachstehenden Komponenten enthält:

  - einen Dreiachsen-Beschleunigungsmesser;
  - ein Dreiachsen-Magnetometer; und/oder
  - ein Dreiachsen-Gyroskop.

9. Hilfsvorrichtung (50) nach einem der Ansprüche 1 bis 8, wobei die Ermittlungseinrichtung (54A, 54B, 54C) zum Ermitteln des Ausrichtungsbezugssystems eine Vorrichtung ist, die zumindest drei optische Marker enthält, die nicht fluchtend ausgerichtet und dazu bestimmt sind, von zumindest einer den Operationsbereich filmenden Kamera sichtbar zu sein.

10. Hilfsvorrichtung (20) nach einem der Ansprüche 1 bis 9, wobei die Kommunikationseinrichtung (25) für das Ausrichtungsbezugssystem eine visuelle Anzeige ist.

11. Hilfsvorrichtung (50; 90) nach einem der Ansprüche 1 bis 10, wobei die Kommunikationseinrichtung (55A, 55B, 55C; 85; 95) für das Ausrichtungsbezugssystem eine drahtgebundene oder drahtlose Verbindung ist.

12. Einheit mit zumindest zwei Hilfsvorrichtungen (20, 20'; 50) nach Anspruch 1, wobei die Hilfsvorrichtungen für Operationsbereiche (ZO) ausgelegt sind, die sich voneinander unterscheiden.

13. Chirurgisches Ausrichtungssystem (SYS1, SYS1'; SYS3) mit zumindest einer Hilfsvorrichtung (20, 20'; 50) nach einem der Ansprüche 1 bis 11 und mit einem chirurgischen Instrument (30; 70), enthaltend:

  - eine Ermittlungseinrichtung (34; 84) zum Ermitteln eines Ausrichtungsbezugssystems (RT) des Instruments; und
  - eine Kommunikationseinrichtung (35; 85) für das Ausrichtungsbezugssystem (RT) des Instruments.

14. Operationssaal (ZS), ausgestattet mit einer chirurgischen Hilfsvorrichtung (20, 20'; 40; 50; 60; 90) nach einem der Ansprüche 1 bis 11 und einer Vorrichtung zum Anzeigen von Bildern und Verarbeiten von Daten (90), enthaltend:

  - einen Bildschirm (91) zum Anzeigen von von dem Operationsbereich (ZO) aufgenommenen Bildern (I);
  - einen Prozessor (92);
  - Einrichtungen zum Eingeben und Bearbeiten von Daten (93); und
  - Einrichtungen (94) zum Empfangen der über die Hilfsvorrichtung kommunizierten Daten.

15. Verfahren zur präoperativen Vorbereitung (P1) einer chirurgischen Operation, umfassend die nachstehenden Schritte:

  - Aufnehmen (S1) zumindest eines dreidimensionalen Bildes (I) eines Operationsbereichs (ZO);
  - Ermitteln (S2) von zumindest drei Bezugspunkten (R1, R2, R3) des Operationsbereichs ausgehend von dem dreidimensionalen Bild;
  - Berechnen (S3) eines Operationsbezugssystems (RO) mittels der Bezugspunkte, wobei das Operationsbezugssystem im Nachhinein von einer Hilfsvorrichtung nach einem der Ansprüche 1 bis 11 erkannt wird; und
  - Ermitteln (S4) von zumindest einem lokalen Bezugssystem ([V]ro) für den durchzuführenden chirurgischen Eingriff.

16. Nichtflüchtiger, computerlesbarer Träger (110), enthaltend ein von einem Computer ausführbares Anweisungsprogramm (11) zum Durchführen des Verfahrens nach Anspruch 15.

**Claims**

1. Ancillary surgical instrument (20, 20'; 50; 90) comprising at least:

   a first contact point (21; 51; 91) intended to come into contact with a first reference point (R1) of an operating area (ZO);
   a second contact point (22; 52; 92) intended to come into contact with a second reference point (R2) of the operating area;
   a tangential contact area (23; 53; 93) intended to come into contact with a third reference point (R3) of the operating area;
   a determination means (24; 54A, 54B, 54C; 84; 94) for determining an orientation reference (RA) of the ancillary instrument in a Galilean orientation reference (RS); and
   a communication means (25; 55A, 55B, 55C; 85; 94) for communicating the determined orientation reference

   characterize in that the ancillary instrument has the form of a Y and comprises:

   at least two branches (26, 27; 56, 57; 96; 97) at the ends of which the first point and the second point (21, 22; 51, 52; 91; 92) are arranged in the form of straight edges;
   a third branch (28; 58; 98) in the form of a handle; and
   a central area having a lower face that forms the tangential contact area (23; 53; 93).

2. Ancillary instrument (20, 20'; 50; 90) according to claim 1, in which the first contact point (21; 51; 91), the second contact point (22; 52; 92), the contact area (23; 53; 93), the determination means (24; 54A, 54B, 54C; 84; 94) and the communication means (25; 55A, 55B, 55C; 85; 94) are incorporated into a single part of the ancillary instrument.

3. Ancillary instrument (20, 20'; 50; 90) according to any one of claims 1 or 2, in which the contact area (23; 53; 93) is a planar tangential area.

4. Ancillary instrument (20, 20'; 50; 90) according to anyone of claims 1 to 3, in which the orientation reference (RA) of the ancillary instrument allows an orientation reference (RO) of the operating area (ZO) to be determined with respect to the Galilean orientation reference (RS) by means of a rotation matrix (Mrors).

5. Ancillary instrument (90) according to claim 1, further comprising validating means (93A) for validating the tangential contact area (93).

6. Ancillary instrument (90) according to claim 5, in which the tangential contact area (93) is transparent and arranged in a cross-ruled form and marked in order to allow a surgeon to determine exactly where the tangential area is in contact with the third reference point.

7. Ancillary instrument (20, 20'; 50; 90) according to anyone of claims 1 to 6, in which the first point (21; 51; 91), the second point (22; 52; 92), and the tangential contact (23; 53; 93) area are coplanar.

8. Ancillary instrument (20'; 50; 90) according to anyone of claims 1 to 7, in which the determination means (24; 84; 94) for determining the orientation reference is a device comprising at least one of the following components:

   a tri-axial accelerometer;
   a tri-axial magnetometer; and/or
   a tri-axial gyroscope.

9. Ancillary instrument (50) according to anyone of claims 1 to 8, in which the determination means (54A, 54B, 54C) for determining the orientation reference is a device comprising at least three non-aligned optical markers, intended to be visible by at least one camera filming the operating area.

10. Ancillary instrument (20) according to anyone of claims 1 to 9, in which the communication means (25) for communicating the orientation reference is a visual display.

11. Ancillary instrument (50; 90) according to anyone of claims 1 to 10, in which the communication means (55A, 55B, 55C; 85; 95) for communicating the orientation reference is a wired or wireless connection.

**12.** A kit comprising at least two ancillary instruments (20, 20'; 50) according to claim 1, the ancillary instruments being designed for operating areas (ZO) that are different from one another.

**13.** Surgical orientation system (SYS1, SYS1'; SYS3) comprising at least one ancillary instrument (20, 20'; 50) according to anyone of claims 1 to 11 and a surgical tool (30; 70) comprising:

a determination means (34; 84) for determining an orientation reference (RT) of the tool; and
a communication means (35; 85) for communicating the orientation reference (RT) of the tool.

**14.** Operating theatre (ZS) equipped with an ancillary surgical instrument (20, 20'; 40; 50; 60; 90) according to anyone of claims 1 to 11 and with a device for displaying images and data processing (90) comprising:

a screen (91) for displaying images (I) taken of the operating area (ZO);
a processor (92);
means for entering and manipulating data (93); and
means for receiving (94) the data communicated by the ancillary instrument.

**15.** Preoperative preparation process (P1) of a surgical operation, comprising the steps of:

taking (S1) at least one three-dimensional image (I) of an operating area (ZO);
determining (S2) at least three reference points (R1, R2, R3) of the operating area from the three-dimensional image;
calculating (S3) an orientation reference (RO) by means of the reference points, the orientation reference being subsequently identified by an ancillary instrument according to anyone of claims 1 to 11; and
determining (S4) at least one local orientation reference ([V]ro) for the surgical operation to be performed.

**16.** Non-transitory medium (110) which can be read by a computer and comprising a program of computer-executable instructions (11) for carrying out the process according to claim 15.

**FIG.1A**

**FIG.1B**

**FIG.2A**

SYS1

-90 -45 0 45 90

**FIG.2B**

**FIG.3A**

**FIG.3B**

FIG.4

EP 3 236 874 B1

FIG.5A

SYS2

A
44
C
45
B'
46
D
A'
B
48
47
R1
41
A1
B1'
43
42
R2
R3
A1'
B1
D'
RO
1

FIG.5B

40
49
47
41
46
42
48
43

17

FIG.7

FIG.6

FIG.8

**FIG.9**

**FIG.10**

**FIG.11**

**FIG.12**

## RÉFÉRENCES CITÉES DANS LA DESCRIPTION

**Documents brevets cités dans la description**

- US 8419746 B
- WO 2006109983 A
- DE 69534862
- WO 2014176207 A
- KR 20060003685
- EP 2719353 A

**Littérature non-brevet citée dans la description**

- **MANBACHI.** *Guided pedicle screw insertion and training*